# EUROPEAN PATENT SPECIFICATION

(11) **EP 3 170 756 B1**
(45) Date of publication and mention of the grant of the patent: **19.09.2018**
(21) Application number: 15195789.1
(22) Date of filing: 23.11.2015
(51) Int. Cl.: B65B 55/08, A61L 2/08, A61L 2/20

(54) **DEVICE AND METHOD FOR STERILIZATION OF A SHEET OF PACKAGING MATERIAL AND FILLING MACHINE**
VORRICHTUNG UND VERFAHREN ZUR STERILISATION EINES VERPACKUNGSMATERIALBLATTS SOWIE FÜLLMASCHINE
DISPOSITIF ET PROCÉDÉ DE STÉRILISATION D'UNE FEUILLE DE MATÉRIAU D'EMBALLAGE ET MACHINE DE REMPLISSAGE

(43) Date of publication of application: 24.05.2017
(73) Proprietor: Tetra Laval Holdings & Finance S.A., 1009 Pully (CH)
(72) Inventor: GHIRARDELLO, Roberto, 41012 Carpi (IT)
(74) Representative: Tetra Pak - Patent Attorneys SE

(56) References cited:
- JP-B2- 3 211 438
- US-A1- 2006 145 093
- US-A1- 2011 062 347

## Description

### Technical field

The invention relates to a device and a method for sterilization of a sheet of packaging material, and a filling machine.

### Background art

In the prior art several different devices and methods for sterilizing packaging material are known, in particular for packaging material intended to form containers. One method widely used in the prior art is sterilization by means of a hydrogen peroxide bath. However, efforts are being made to reduce the use of chemicals when sterilizing the packaging material. Therefore, devices and methods have also become known which sterilize packaging material by means of radiation, in particular by means of electron beams. In general, there is provided an emitter that is adapted to emit charge carriers, in particular electrons, wherein the packaging material can be sterilized by the charge carriers. For this purpose the packaging material and the emitter or the emitters, respectively, are moved relatively to each other.

Such a device is shown, for example, in US2006/145093 A1. This type of sterilization by means of radiation is conventionally conducted in a radiation chamber of a sterilization device. The radiation chamber itself needs to be sterilized before it can be used for sterilization of the packaging material, normally upon production start every morning. In order to sterilize the radiation chamber, a flow of vapour of hydrogen peroxide is introduced into the radiation chamber.

The need is felt to limit or completely avoid emission of the vapour of hydrogen peroxide into the surroundings of the sterilization devices.

### Summary of the invention

It is an object of the present invention to mitigate, alleviate or eliminate one or more of the above-identified deficiencies in the art and disadvantages singly or in any combination and solve at least the above mentioned problem.

According to a first aspect of the invention, these and other objects are achieved in full, or at least in part, by a device for sterilization of a sheet of packaging material, particularly a device for sterilization of a sheet of packaging material by means of electron beam. The device comprises a first chamber having an inlet and an outlet for the sheet of packaging material, and a second chamber having an inlet and an outlet for the sheet of packaging material. The outlet of the first chamber is connected to the inlet of the second chamber. The first chamber comprises an element which is movable between a first position, in which the device is in a mode for sterilization of the sheet of packaging material, and a second position, in which the inlet of the first chamber of the device is substantially closed and the device is in a mode for sterilization of the second chamber of the device. In this way, the inlet of the device, i.e. the inlet of the first chamber of the device, is prevented from leaking vapour of hydrogen peroxide out of the device and into the surrounding environment during sterilization of the second chamber of the device. This is very important since there are normally regulations in this regards which, among other things, include a maximum allowed value of the amount of the vapour of hydrogen peroxide in the surrounding environment of the device.

In a preferred embodiment of the present invention, the movable element may be constituted by a flap which is moved by means of an actuator device, particularly a pneumatic cylinder. This is advantageous in that it is a simple but reliable solution on how to open and close the inlet of the first chamber.

The second chamber may further comprise a vapour inlet through which the vapour of hydrogen peroxide is introduced when the second chamber is to be sterilized, i.e. when the movable element is in its second position.

The first chamber may further comprise a vapour outlet through which vapour is sucked out of the device by means of a suction pipe which is connected to the vapour outlet.

When the device is operated for sterilizing the sheet of packaging material, the movable element is arranged in its first position so that the inlet of the suction chamber is open.

When the second chamber of the device is to be sterilized, the movable element is arranged in its second position so that the inlet of the first chamber is closed. In this configuration, the second chamber is sterilized and the vapour of hydrogen peroxide is sucked away through the vapour outlet and the suction pipe.

The first chamber may be a suction chamber and the second chamber may be an electron beam irradiation chamber, particularly a low voltage electron beam irradiation chamber. It is in the radiation chamber that the sterilization of the sheet of packaging material takes place by using a low voltage electron beam. Vapour of hydrogen peroxide is preferably used for sterilization of the radiation chamber of the device. This process is conducted when the device is in its mode for sterilization of the radiation chamber, and thus the movable element is in its second position to prevent vapour of hydrogen peroxide from escaping out of the inlet of the suction chamber.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of the element, device, component, means, step, etc., unless explicitly stated otherwise.

### Brief description of the drawings

The above objects, as well as additional objects, features and advantages of the present invention, will be more fully appreciated by reference to the following illustrative and non-limiting detailed description of preferred embodiments of the present invention, when taken in conjunction with the accompanying drawings, wherein:
Figure 1 is a cross sectional view of an exemplary embodiment of a device according to the invention when in a mode for sterilization of a sheet of packaging material.
Figure 2 is a cross sectional view of the device when in a mode for sterilization of the second chamber of the device.
Figure 3 is a cross sectional view of the second chamber of the device.
Figure 4 is an enlarged view of a portion of the device in Figure 1 and Figure 2 and of a movable element arranged within the device.
Figure 5 is a block diagram of an exemplary embodiment of a method for sterilization of a sheet of packaging material according to the invention.

### Detailed description of preferred embodiments of the invention

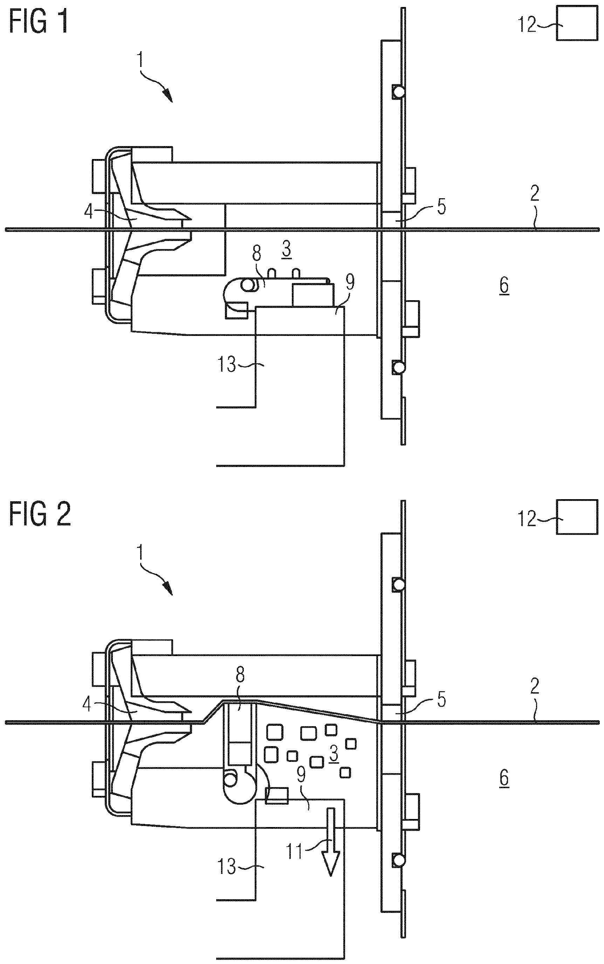

Figure 1 and Figure 2 illustrate a device 1 for sterilization of a sheet of packaging material 2 with electron beam according to an exemplary embodiment of the invention. The device 1 has a first chamber 3 which is constituted by a suction chamber and a second chamber 6 which is constituted by a radiation chamber, i.e. a sterilization chamber. The suction chamber 3 has an inlet 4 and an outlet 5, and the radiation chamber 6 has an inlet 5 and an outlet. The outlet 5 of the suction chamber 3 is connected to the inlet 5 of the radiation chamber 6. Thus, the sheet of packaging material 2 can be introduced from the exterior of the device 1 into the suction chamber 3 via its inlet 4, travel through the suction chamber 3 and out of the outlet 5 into the radiation chamber 6 via its inlet 5. The suction chamber 3 further comprises a vapour outlet 9 which is connected to a suction pipe 13, and a movable element 8. The movable element 8 is constituted by a flap which is movable between a first position (Figure 1), in which the device 1 is in a mode for sterilization of the sheet of packaging material 2 (conducted in the radiation chamber 6), and a second position (Figure 2), in which the inlet 4 of the suction chamber 3 is substantially closed and the device 1 is in a mode for sterilization of the radiation chamber 6.

When the device 1 is in the mode for sterilization of the sheet of packaging material 2, the sheet of packaging material 2 travels through the device 1 and is sterilized by means of a low voltage electron beam (LVEB) device 15. Here, the flap 8 is arranged in its first position (Figure 1). However, when the radiation chamber 6 needs to be sterilized (normally upon start of the device 1 in the morning), the flap 8 is placed in its second position and the inlet 4 of the suction chamber 3 is thereby substantially closed. Thereafter, vapour of hydrogen peroxide is introduced directly into the radiation chamber 6 through a vapour inlet 12 to sterilize the chamber 6. Simultaneously, the vapour outlet 9 in the suction chamber 3 provides suction via the suction pipe 13 which is connected to a vacuum. This is done in order to suck the residues of the vapour of hydrogen peroxide out of the radiation chamber 6. Further, since the flap 8 seals off the inlet 4 of the suction chamber 3, the residues of the vapour of hydrogen peroxide cannot escape out that way and into the surroundings of the device 1.

As stated above, the vapour used for sterilizing the radiation chamber 6 of the device 1 is preferably hydrogen peroxide. Hydrogen peroxide is a very effective gas for sterilization, but has to be handled carefully. The flap 8 has the purpose of sealing the inlet 4 of the suction chamber 3, when it is in its second position (Figure 2), so that the gas is prevented from exiting the device 1 during the sterilization of the radiation chamber 6, or at least so that only an acceptable amount of gas is allowed to exit the device 1 during sterilization of the radiation chamber 6.

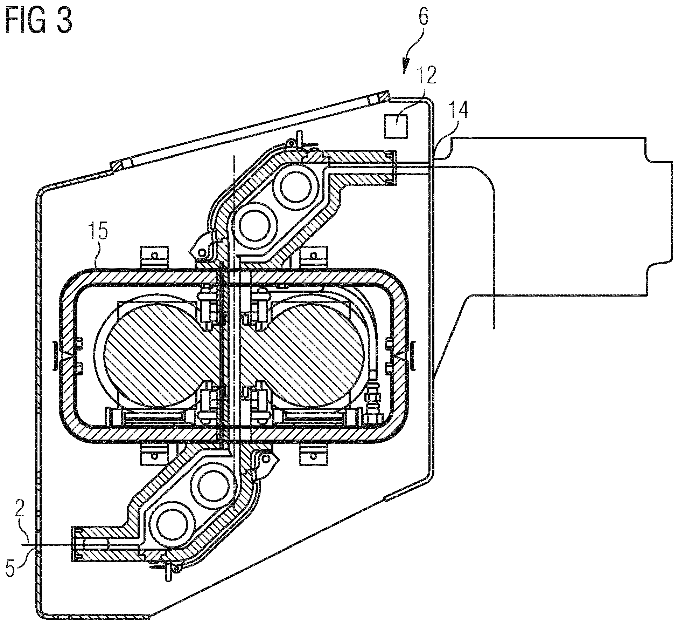

Figure 3 illustrates the radiation chamber 6 of the device 1. As stated above, the radiation chamber 6 has an inlet 5 and an outlet 14 for the sheet of packaging material 2. The sheet of packaging material 2 enters into the radiation chamber 6 through the inlet 5, passes the low voltage electron beam (LVEB) device 15 where it is sterilized on both sides by electron beam emitters, and exits the radiation chamber 6 through the outlet 14. There is a constant overpressure present in the radiation chamber 6 so that dirty, i.e. unsterile, air is prevented from entering into the radiation chamber 6 during the sterilization of the sheet of packaging material 2 and also during sterilization of the radiation chamber 6.

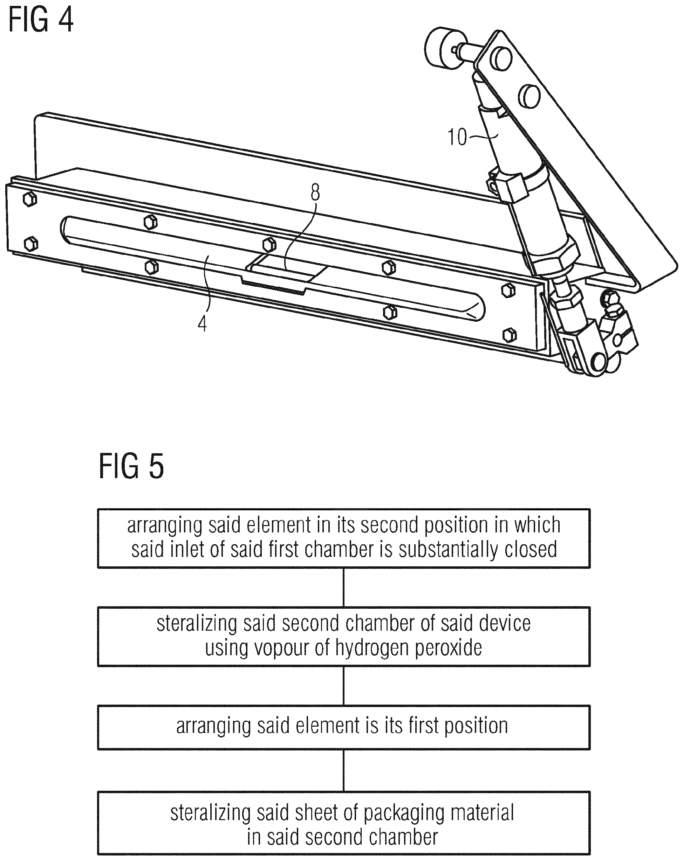

Figure 4 is a perspective view of a portion of the device 1. Here, the flap 8 and the inlet 4 of the second chamber 3 are specifically illustrated. In Figure 4, it can be seen that the flap 8 is operated and moved by means of a pneumatic cylinder 10.

Figure 5 is a block diagram illustrating the steps of the method of sterilizing first the radiation chamber and then the packaging material in the device of Figures 1 to 4.

Firstly, upon start of the device, the flap is arranged in its second position. Here, the inlet of the suction chamber is substantially closed. Now, the radiation chamber can be sterilized by means of vapour of hydrogen peroxide entering through the vapour inlet. Thereafter, the flap is arranged in its first position so that the device is in the mode for sterilization of the sheet of packaging material. And finally, the sheet of packaging material is sterilized in the radiation chamber.

It is understood that other variations in the present invention are contemplated and in some instances, some features of the invention can be employed without a corresponding use of other features. Accordingly, it is appropriate that the appended claims be construed broadly in a manner consistent with the scope of the invention.

## Claims

1. Device for sterilization of a sheet of packaging material (2), said device (1) comprising
a first chamber (3) having an inlet (4) and an outlet (5) for said sheet of packaging material (2), and
a second chamber (6) having an inlet (5) and an outlet for said sheet of packaging material (2), **characterised in that** said outlet (5) of said first chamber (3) is connected to said inlet (5) of said second chamber (6),
wherein said first chamber (3) comprises an element (8) which is movable between a first position, in which said device (1) is in a mode for sterilization of said sheet of packaging material (2), and a second position, in which said inlet (4) of said first chamber (3) is substantially closed and said device (1) is in a mode for sterilization of said second chamber (6).

2. The device (1) according to claim 1, wherein said movable element (8) comprises a flap.

3. The device (1) according to claim 2, wherein said flap (8) is moved by means of a pneumatic cylinder (10) .

4. The device (1) according to any one of the preceding claims, wherein said first chamber (3) further comprises a vapour outlet (9) through which vapour is sucked out of the device (1).

5. The device (1) according to claim 4, wherein a suction pipe (13) is connected to said vapour outlet (9).

6. The device (1) according to any one of the preceding claims, wherein said second chamber (6) further comprises a vapour inlet (12).

7. The device (1) according to any one of the preceding claims, wherein said first chamber (3) is a suction chamber and said second chamber (6) is an electron beam irradiation chamber.

8. The device (1) according to any one of the preceding claims, wherein vapour of hydrogen peroxide is used for sterilization of said second chamber (6) when said device (1) is in said mode for sterilization of said second chamber (6), and said element (8), being in its second position, prevents said vapour of hydrogen peroxide from escaping out of said inlet (4) of said first chamber (3).

9. A filling machine for filling a packaging material (2) with a food product, comprising a device (1) according to any one of claims 1 to 8 for sterilization of said sheet of packaging material (2) .

10. A method for sterilizing a sheet of packaging material by means of a sterilization device, said device comprising a first chamber having an inlet and an outlet for said packaging material and an element movable between a first position and a second position, and a second chamber having an inlet and an outlet for said packaging material, said outlet of said first chamber being connected to said inlet of said second chamber, wherein said method comprises the steps of
arranging said element in its second position, in which said inlet of said first chamber is substantially closed,
sterilizing said second chamber of said device using vapour of hydrogen peroxide,
arranging said element in its first position, and sterilizing said sheet of packaging material in said second chamber.

## Patentansprüche

1. Vorrichtung zur Sterilisation eines Verpackungsmaterialblattes (2), wobei die Vorrichtung (1) umfasst:
eine erste Kammer (3) mit einem Einlass (4) und einem Auslass (5) für das Verpackungsmaterialblatt (2), und
eine zweite Kammer (6) mit einem Einlass (5) und einem Auslass für das Verpackungsmaterialblatt (2), **dadurch gekennzeichnet, dass**
der Auslass (5) der ersten Kammer (3) mit dem Einlass (5) der zweiten Kammer (6) verbunden ist,
wobei die erste Kammer (3) ein Element (8) umfasst, das zwischen einer ersten Position, in der die Vorrichtung (1) in einem Modus zur Sterilisation des Verpackungsmaterialblattes (2) ist, und einer zweiten Position beweglich ist, in der der Einlass (4) der ersten Kammer (3) im Wesentlichen geschlossen ist und die Vorrichtung (1) in einem Modus zur Sterilisation der zweiten Kammer (6) ist.

2. Vorrichtung (1) nach Anspruch 1, wobei das bewegliche Element (8) eine Klappe einschließt.

3. Vorrichtung (1) nach Anspruch 2, wobei die Klappe (8) mittels eines pneumatischen Zylinders (10) bewegt wird.

4. Vorrichtung (1) nach einem der vorhergehenden Ansprüche, wobei die erste Kammer (3) ferner einen Dampfauslass (9) umfasst, durch den Dampf aus der Vorrichtung (1) herausgesaugt wird.

5. Vorrichtung (1) nach Anspruch 4, wobei ein Saugrohr (13) mit dem Dampfauslass (9) verbunden ist.

6. Vorrichtung (1) nach einem der vorhergehenden Ansprüche, wobei die zweite Kammer (6) ferner einen Dampfeinlass (12) umfasst.

7. Vorrichtung (1) nach einem der vorhergehenden Ansprüche, wobei die erste Kammer (3) eine Saugkammer ist und die zweite Kammer (6) eine Elektronenstrahlbestrahlungskammer ist.

8. Vorrichtung (1) nach einem der vorhergehenden Ansprüche, wobei Wasserstoffperoxiddampf zur Sterilisation der zweiten Kammer (6) verwendet wird, wenn die Vorrichtung (1) in dem Modus zur Sterilisation der zweiten Kammer (6) ist, und das Element (8), welches sich in seiner zweiten Position befindet, das Entweichen des Wasserstoffperoxiddampfes aus dem Einlass (4) der ersten Kammer (3) verhindert.

9. Füllmaschine zum Füllen eines Verpackungsmaterials (2) mit einem Nahrungsmittelprodukt, umfassend eine Vorrichtung (1) gemäß einem der Ansprüche 1 bis 8 zur Sterilisation des Verpackungsmaterialblattes (2).

10. Verfahren zum Sterilisieren eines Verpackungsmaterialblattes mittels einer Sterilisationsvorrichtung, wobei die Vorrichtung eine erste Kammer mit einem Einlass und einem Auslass für das Verpackungsmaterial und einem Element, das zwischen einer ersten Position und einer zweiten Position beweglich ist, und eine zweite Kammer mit einem Einlass und einem Auslass für das Verpackungsmaterial umfasst, wobei der Auslass der ersten Kammer mit dem Einlass der zweiten Kammer verbunden ist, wobei das Verfahren die Schritte umfasst:
Anordnen des Elements in seiner zweiten Position in der der Einlass der ersten Kammer im Wesentlichen geschlossen ist,
Sterilisieren der zweiten Kammer der Vorrichtung unter Verwendung von Wasserstoffperoxiddampf,
Anordnen des Elements in seiner ersten Position, und Sterilisieren des Verpackungsmaterialblattes in der zweiten Kammer.

## Revendications

1. Dispositif pour la stérilisation d'une feuille de matériau d'emballage (2), ledit dispositif (1) comprenant
une première chambre (3) ayant une entrée (4) et une sortie (5) pour ladite feuille de matériau d'emballage (2), et
une deuxième chambre (6) ayant une entrée (5) et une sortie pour ladite feuille de matériau d'emballage (2),
**caractérisé en ce que**
ladite sortie (5) de ladite première chambre (3) est reliée à ladite entrée (5) de ladite deuxième chambre (6),
dans lequel ladite première chambre (3) comprend un élément (8) qui est mobile entre une première position, dans laquelle ledit dispositif (1) est dans un mode de stérilisation de ladite feuille de matériau d'emballage (2), et une deuxième position, dans laquelle ladite entrée (4) de ladite première chambre (3) est sensiblement fermée et ledit dispositif (1) est dans un mode de stérilisation de ladite deuxième chambre (6).

2. Dispositif (1) selon la revendication 1, dans lequel ledit élément mobile (8) comprend un rabat.

3. Dispositif (1) selon la revendication 2, dans lequel ledit rabat (8) est déplacé au moyen d'un vérin pneumatique (10).

4. Dispositif (1) selon l'une quelconque des revendications précédentes, dans lequel ladite première chambre (3) comprend en outre une sortie de vapeur (9) par laquelle de la vapeur est aspirée hors du dispositif (1).

5. Dispositif (1) selon la revendication 4, dans lequel un tuyau d'aspiration (13) est relié à ladite sortie de vapeur (9).

6. Dispositif (1) selon l'une quelconque des revendications précédentes, dans lequel ladite deuxième chambre (6) comprend en outre une entrée de vapeur (12).

7. Dispositif (1) selon l'une quelconque des revendications précédentes, dans lequel ladite première chambre (3) est une chambre d'aspiration et ladite deuxième chambre (6) est une chambre d'irradiation par faisceau d'électrons.

8. Dispositif (1) selon l'une quelconque des revendications précédentes, dans lequel de la vapeur de peroxyde d'hydrogène est utilisée pour la stérilisation de ladite deuxième chambre (6) quand ledit dispositif (1) est dans ledit mode de stérilisation de ladite deuxième chambre (6), et ledit élément (8), qui est dans sa deuxième position, empêche ladite vapeur de peroxyde d'hydrogène de s'échapper par ladite entrée (4) de ladite première chambre (3).

9. Machine de remplissage destinée à remplir un matériau d'emballage (2) avec un produit alimentaire, comprenant un dispositif (1) selon l'une quelconque des revendications 1 à 8 pour la stérilisation de ladite feuille de matériau d'emballage (2).

10. Procédé de stérilisation d'une feuille de matériau d'emballage au moyen d'un dispositif de stérilisation, ledit dispositif comprenant une première chambre ayant une entrée et une sortie pour ledit matériau d'emballage et un élément mobile entre une première position et une deuxième position, et une deuxième chambre ayant une entrée et une sortie pour ledit matériau d'emballage, ladite sortie de ladite première chambre étant reliée à ladite entrée de ladite deuxième chambre, ledit procédé comprenant les étapes consistant à
placer ledit élément dans sa deuxième position, dans laquelle ladite entrée de ladite première chambre est sensiblement fermée,
stériliser ladite deuxième chambre dudit dispositif en utilisant de la vapeur de peroxyde d'hydrogène,
placer ledit élément dans sa première position, et stériliser ladite feuille de matériau d'emballage dans ladite deuxième chambre.
